# EUROPÄISCHE PATENTSCHRIFT

(11) **EP 3 275 362 B1**
(45) Veröffentlichungstag und Bekanntmachung des Hinweises auf die Patenterteilung: **28.12.2022**
(21) Anmeldenummer: 16181681.4
(22) Anmeldetag: 28.07.2016
(51) Int. Cl.: A61B 5/00, A61B 5/0205, A61B 5/145, A61B 5/1486, G06F 1/16

(54) **VORRICHTUNG ZUR ÜBERWACHUNG EINES ZUSTANDS EINES LEBEWESENS**
DEVICE FOR MONITORING A CONDITION OF A LIVING BEING
DISPOSITIF DE SURVEILLANCE D'UN ETAT D'UN ETRE VIVANT

(43) Veröffentlichungstag der Anmeldung: 31.01.2018
(73) Patentinhaber: Fraunhofer-Gesellschaft zur Förderung der angewandten Forschung e.V., 80686 München (DE)
(72) Erfinder: Bertsch, Thomas, 68723 Schwetzingen (DE); Langenstein, Bernd, 90425 Nürnberg (DE); Jank, Michael, 91058 Erlangen (DE); Oertel, Susanne, 96175 Pettstadt (DE); Lang, Nadine Ramona, 91054 Erlangen (DE); Struck, Matthias, 90766 Fürth (DE); Renner, Esther Ann, 91058 Erlangen (DE); Hofmann, Christian, 90425 Nürnberg (DE)
(74) Vertreter: Schenk, Markus

(56) Entgegenhaltungen:
- WO-A1-2016/112248
- KR-A- 20100 137 306
- US-A1- 2006 078 873
- US-A1- 2007 135 698
- US-A1- 2008 023 325
- US-A1- 2008 281 178
- US-A1- 2010 145 317
- US-A1- 2014 024 915
- US-A1- 2015 112 165
- US-A1- 2015 245 777
- US-A1- 2015 277 572
- US-A1- 2015 335 254
- US-A1- 2016 168 613
- SUSANNE OERTEL ET AL: "Screen-printed Biochemical Sensors for Detection of Ammonia Levels in Sweat - Towards Integration with Vital Parameter Monitoring Sports Gear :", PROCEEDINGS OF THE 9TH INTERNATIONAL JOINT CONFERENCE ON BIOMEDICAL ENGINEERING SYSTEMS AND TECHNOLOGIES, 1 January 2016 (2016-01-01), pages 160-165, XP055471623, DOI: 10.5220/0005691501600165 ISBN: 978-989-7581-70-0

## Beschreibung

Die Erfindung bezieht sich auf eine Vorrichtung zur Überwachung eines Zustands eines Lebewesens. Der Zustand umfasst beispielsweise mehrere Vitalwerte des Lebewesens oder z. B. einen Belastungszustand. Dabei ist das Lebewesen beispielsweise ein Mensch oder ein Tier. Weiterhin bezieht sich die Erfindung auf ein Verfahren zur Überwachung eines Zustands eines Lebewesens.

Im modernen Stand der Technik gibt es viele tragbare Computer oder tragbare Sensoren, die von einem Benutzer direkt am Körper, z. B. als Kleidungsstück getragen werden können. Solche "Wearables" erlauben z. B. die Überwachung des Herzens eines Menschen. Auch ist es bekannt, Biosensoren zu verwenden, die z. B. den Laktatwert im Schweiß eines Menschen ermitteln (siehe z. B. [1]). Solche Laktatmessungen sind dabei der Leistungsdiagnostik bei sportmedizinischen Untersuchungen entlehnt. Dabei wird klassisch der Laktatwert invasiv ermittelt, wofür der Sportler eine Pause einlegen muss.

Für nicht-invasive Messungen finden sich Ansätze im Stand der Technik. So soll Schweiß am Handgelenk mit einer Art von Armband analysiert werden, siehe z. B. [2]. Weiterhin ist es auch bekannt, Elektroden von Sensoren (z. B. WO 2011/156095 A2) oder Komponenten von elektronischen Bauteilen (z. B. US 2014/0024915 A1) in Textilien einzubringen.

Die Druckschrift US 2015/0277572 A1 offenbart eine Anzeigevorrichtung in Form eines Armbandes, wobei die Anzeige in Abhängigkeit von Bewegungsdaten des Armbandes gesteuert wird, wobei zusätzliche Sensoren beispielsweise die Bestandteile von Schweiß analysieren können. Diese Sensoren sind hier Teil des Armbandes und müssen nicht zu einer Auswertvorrichtung übertragen werden.

Die Druckschrift US 2015/0112165 A1 offenbart Geräte, die Schweiß wahrnehmen und dabei eine chronologische Erfassung ermöglichen, wobei eine Einbettung eines Schweißratensensors in ein Messgerät erfolgt. Die Sensoren bilden hier mit der Auswertvorrichtung eine Einheit.

Die Druckschrift US 2008/0023325 A1 offenbart eine nanobasierte Sensorvorrichtung, die eine Arbeitselektrode im Nanomaßstab umfasst, die zum ultraempfindlichen Nachweis von bestimmten Zielmolekülen verwendet werden kann.

Die Druckschrift "Screen-printed Biochemical Sensors for Detection of Ammonia Levels in Sweat - Towards Integration with Vital Parameter Monitoring Sports Gear / Autoren: Susanne Oertel, Michael P. M. Jank, Lothar Frey, Christian Hofmann, Nadine Lang und Matthias Struck" offenbart eine Vorrichtung zum Überwachen eines Fitness-Zustands eines Trainierenden, wobei u.a. Ammoniak im Schweiß eines Sportlers gemessen wird.

Die Aufgabe der Erfindung besteht darin, eine Vorrichtung zur Überwachung eines Zustands eines Lebewesens vorzuschlagen, die eine Alternative zum Stand der Technik darstellen.

Die Erfindung löst die Aufgabe durch eine Vorrichtung zur Überwachung eines Zustands eines Lebewesens. Die Vorrichtung weist mindestens einen ersten Sensor, einen zweiten Sensor und eine Auswertevorrichtung auf. Der erste Sensor ist derartig ausgestaltet, basierend auf einem Anteil von Ammoniak im Schweiß des Lebewesens nicht-invasiv einen Messwert zu erzeugen. Der zweite Sensor ist derartig ausgestaltet, einen Messwert bezüglich der Atmung oder der Herztätigkeit des Lebewesens zu erzeugen. Schließlich ist die Auswertevorrichtung derartig ausgestaltet, basierend auf dem Messwert des ersten Sensors und dem Messwert des zweiten Sensors ein skalares Maß für eine Belastung des Lebewesens zu erzeugen. Das skalare Maß ergibt sich dabei aus den mindestens zwei Messwerten und ist in einer Ausgestaltung eine Kombination von Werten oder nur ein einzelner Wert und ist in einer weiteren Ausgestaltung eine Art von genereller Aussage über einen Belastungszustand des zu untersuchenden Lebewesens. So erlaubt das skalare Maß in einer Ausgestaltung eine Aussage, ob die Belastung beibehalten oder reduziert werden sollte. Praktisch bedeutet dies in einer Anwendung, dass das Lebewesen eine (z. B. sportliche) Aktivität vermindern sollte oder in Bezug auf die Anstrengung beibehalten kann. Insgesamt werden somit wenigstens zwei Messwerte von zwei unterschiedlichen Messgrößen zu einem skalaren Maß zusammengefasst. Dieses Maß beschreibt dabei die Belastung des Lebewesens als einen Zustand des Lebewesens.

Um das Maß zu ermitteln, werden in einer Ausgestaltung Datensätze herangezogen, bei denen die durch die von der Vorrichtung nicht-invasiv ermittelten Messergebnisse mit invasiv ermittelten Messwerten abgeglichen sind. So wird beispielsweise bei den Messungen für die Datensätze der Laktatwert invasiv aus dem Blut als Maß für die Belastung ermittelt. Dies geschieht vorzugweise bei einer ausreichend großen Menge an Probanden, um Datensätze zu erhalten, die die Abbildung von mindestens zwei Werten (z. B. Ammoniakanteil und Atmung oder Ammoniakanteil und Herzschlag) auf das skalare Maß für die Belastung des Lebewesens erlaubt. Die Datensätze bestehen somit beispielsweise aus Tabellen, Algorithmen und/oder Formeln.

Die Vorrichtung erlaubt insbesondere eine schnelle, kontinuierliche, nicht-invasive, d. h. insbesondere blutlose, Vitalparametermessung, die in einer weiteren Ausgestaltung zu einer Zustandseinschätzung erweitert wird. Dabei findet vorzugsweise eine direkte Datenauswertung von Messwerten statt.

Bei der erfindungsgemäßen Vorrichtung wird über den ersten Sensor Ammoniak (chemische Bezeichnung: NH₃) gemessen. Ammoniak entsteht innerhalb des Purin-Nukleotid-Zyklus. In der Leistungsdiagnostik wurde der Ammoniakgehalt im Blut bereits untersucht und als aussagekräftig anerkannt [8].

Dies ist ein Vorteil gegenüber der Laktatmessung, für die in den letzten Jahren eindeutig belegt wurde, dass die Leistungsdiagnostik über den Laktatwert nur durch eine Blutuntersuchung, d. h. durch einen invasiven Eingriff, aussagekräftig ist. Mehrfach wurde nachgewiesen, dass die Konzentration des Laktats im Schweiß nicht mit der Konzentration im Blut korreliert. Vielmehr produzieren die Schweißdrüsen selbst Laktat und machen somit eine eindeutige Diagnostik über den Schweiß unmöglich. Im Gegensatz dazu konnte belegt werden, dass Ammoniak, das sich im Schweiß befindet, hauptsächlich aus dem Blut ausgewaschen wird [3 - 6]. Aus Versuchen lässt sich darauf schließen, dass ein physiologischer Zusammenhang zwischen einem Blutwert und Schweißwert gegeben ist.

Die erfindungsgemäße Vorrichtung erlaubt die Überwachung eines Lebewesens, wobei die Vorrichtung - in einer Ausgestaltung sogar - am Körper getragen werden kann und neben der eigentlichen Messung auch eine direkte Auswertung und vorzugsweise in Bezug auf Referenzwerte auch eine Bewertung der Messwerte vornimmt.

Die Messungen gelten dabei einerseits dem Ammoniakanteil im Schweiß sowie andererseits mindestens der Atmung oder der Herztätigkeit. Dabei werden in einer Ausgestaltung Atmung und Herztätigkeit durch entsprechend viele zweite Sensoren vermessen. In einer Ausgestaltung sind somit mehrere zweite Sensoren vorhanden, wobei ein zweiter Sensor einen Messwert bezüglich der Atmung und ein anderer zweiter Sensor einen Messwert bezüglich der Herztätigkeit erzeugt. Ergänzend werden noch weitere Messwerte erzeugt, die sich z. B. auf andere Komponenten des Schweißes beziehen.

Eine Messanordnung zur Messung von Schweiß zeigt beispielsweise die JP 2006-43120 A2 oder die US 2014/0012114 A1. Die Messung von Ammoniak in Flüssigkeiten ist beispielsweise bekannt aus der US 4,700,709 A.

Die Auswertevorrichtung korreliert somit in einer Ausgestaltung den Messwert des ersten Sensors mit dem Messwert des mindestens einen zweiten Sensors, der sich auf etablierte Vitalparametern, wie dem EKG (Herzrate und Herzratenvarabilität) und/oder die Atemrate bezieht.

In einer Ausgestaltung werden insbesondere von der Vorrichtung alle Messwerte nicht-invasiv ermittelt. Dies erleichtert die Anwendung der Vorrichtung und verhindert auch mögliche Komplikationen für das Lebewesen.

In einer Ausgestaltung ist vorgesehen, dass der erste Sensor und der zweite Sensor in einer an einem Körper des Lebewesens tragbaren Trägervorrichtung angeordnet sind. Die Trägervorrichtung ist z. B. ein textiles Kleidungstück oder eine Art von Uhr bzw. Armband oder eine andere Art eines Bandes, das um ein oder an einem Körperteil getragen werden kann.

In einer Ausgestaltung ist die Auswertevorrichtung separat von den Sensoren und der Trägervorrichtung angeordnet und ist z. B. per Funk mit dem ersten und dem zweiten Sensor verbunden.

In einer weiteren Ausgestaltung sind der erste Sensor, der zweite Sensor und die Auswertevorrichtung in einer solchen an einem Körper des Lebewesens tragbaren Trägervorrichtung angeordnet. In dieser Ausgestaltung ist daher die Vorrichtung komplett in einer Trägervorrichtung, um von dem Lebewesen am Körper getragen zu werden.

In einer Ausgestaltung ist die Auswertevorrichtung derartig ausgestaltet, dass sie Bewegungsartefakte kompensiert.

In einer Anwendung handelt es sich bei dem zu überwachenden Zustand des Lebewesens insbesondere um Belastungszustände, z. B. während einer sportlichen Betätigung oder während einer medizinischen Behandlung.

In einer Ausgestaltung ist vorgesehen, dass die Vorrichtung mehrere erste Sensoren aufweist. In einer Ausgestaltung befinden sich die ersten Sensoren an unterschiedlichen Positionen, so dass der Schweiß an unterschiedlichen Stellen des Lebewesens analysiert werden kann. Somit lassen sich beispielsweise gezielt unterschiedliche Muskelpartien untersuchen. Weiterhin sind in einer Ausgestaltung die ersten Sensoren derartig angeordnet, dass sich Bewegungsartefakte in der Messung weitgehend kompensieren. In anderen Ausgestaltungen wird durch die mehreren ersten Sensoren Redundanz geschaffen, so dass auch ein interner Abgleich der ersten Sensoren möglich wird. In einer Ausgestaltung sind die ersten Sensoren gleich ausgeführt. In einer alternativen Ausgestaltung unterscheiden sich zumindest zwei erste Sensoren voneinander. In einer Ausgestaltung ist ein zusätzlicher Sensor vorhanden, der einen Messwert für einen Anteil einer von Ammoniak verschiedenen Komponente (z. B. Laktat oder eines Elektrolyten) des Schweißes erzeugt.

Eine Ausgestaltung besteht darin, dass die Vorrichtung mindestens eine Transportvorrichtung aufweist. Dabei ist die Transportvorrichtung derartig ausgestaltet, den Schweiß hin zu dem ersten Sensor und/oder fort von dem ersten Sensor zu transportieren. Eine Transportvorrichtung erlaubt es in einer Ausgestaltung, den ersten Sensor abgesetzt vom direkten Kontaktbereich mit dem Lebewesen zu platzieren. Alternativ oder ergänzend wird durch die Transportvorrichtung ein im Wesentlichen kontinuierlicher Fluss des Messmediums Schweiß ermöglicht und damit jeweils aktuelle Messungen gewährleistet.

In einer Ausgestaltung ist vorgesehen, dass die Transportvorrichtung mindestens eine mit mindestens einem Kanal versehene Komponente aufweist. Die Komponente kann daher auch als Führungskomponente verstanden werden, die in z. B. mikrostrukturierten Kanälen oder Kapillaren den Schweiß zum ersten Sensor bzw. von diesem fort führt.

Eine Ausgestaltung besteht darin, dass die Transportvorrichtung mindestens eine ein saugfähiges Material aufweisende Komponente aufweist. In einer Ausgestaltung weist die Komponente Materialien mit unterschiedlicher Saugfähigkeit auf.

In einer Ausgestaltung ist vorgesehen, dass die Transportvorrichtung mindestens eine Pumpvorrichtung aufweist. Eine solche Pumpe ist dabei vorzugsweise eine Mikropumpe, die den Schweiß passend bewegt.

Eine Ausgestaltung besteht darin, dass der erste Sensor mindestens eine auf einer Folie gedruckte ionenselektive Elektrode aufweist.

Eine ionenselektive Elektrode (andere Bezeichnungen sind: ionenspezifische oder ionensensitive Elektrode) erlaubt die Messung der Konzentration bzw. der Aktivität eines bestimmten gelösten Ions. Hierfür wird die ionenselektive Elektrode und eine zweite Elektrode - die Bezugs- bzw. Referenzelektrode - in eine Messlösung eingebracht und die Spannung zwischen den Elektroden gemessen.

Die ionenselektive Elektrode ist dabei in einer Ausgestaltung insbesondere derartig ausgestaltet, dass sie Ionen misst, die die Bestimmung des Ammoniaks erlauben. In einer Ausgestaltung erlaubt daher die ionenselektive Elektrode die Messung des Kations Ammonium (chemische Bezeichnung NH₄⁺).

Ausgehend davon, dass im Schweiß unterschiedliche Ionen mit ähnliche Eigenschaften auftreten, ist in einer Ausgestaltung die Auswertevorrichtung derartig ausgeführt, dass sie bei der Auswertung der Messwerte des ersten Sensors die Anwesenheit von unterschiedlichen Ionen berücksichtigt.

Die Aufbringung auf eine - insbesondere - flexible Folie hilft dabei der Unterbringung in dem besagten Trägervorrichtung und behindert nicht den Tragekomfort.

In einer Ausgestaltung ist die Folie durchsichtig.

In einer weiteren Ausgestaltung besteht die Folie zumindest teilweise aus Polyester (PET oder PEN) oder aus Polyimiden (PI) oder anderen synthetischen Materialien wie Polyurethan (PU) oder aus synthetischen Textilien.

In einer Ausgestaltung ist vorgesehen, dass der erste Sensor eine ionenselektive Membran, eine Referenzelektrode und eine Gegenelektrode aufweist. Die Membran lässt dabei insbesondere die zu messenden Ionen passieren.

In einer Ausgestaltung ist die Membran derartig ausgeführt, dass sie Ammonium-Ionen (NH₄⁺) passieren lässt. Da eine solche Membran beispielsweise auch Kationen wie K⁺ oder Na⁺ passieren lässt, stehen in einer Ausgestaltung der Auswertevorrichtung entsprechende Kalibrierungsdaten zur Verfügung, um eine solche Beeinträchtigung der Messwerte zu kompensieren.

Insgesamt ist der erste Sensor vorzugweise klein, flexibel, tragbar, hautverträglich, textilintegrierbar, kostengünstig und energieeffizient ausgestaltet.

Vorzugweise handelt es sich um einen gedruckten ersten Sensor, der gedruckte Arbeits- und Referenzelektroden sowie mindestens eine Passivierungsschicht aufweist.

Die Herstellung erfolgt vorzugsweise mit kommerziell erhältlichen Siebdruckpasten, wobei in einer Ausgestaltung eine Optimierung der gedruckten Elektroden mittels Ausheizen bei pastenspezifischen Temperaturen erfolgt. In einer Ausgestaltung sieht die Fertigstellung der Referenzelektrode das Aufbringen einer Mischung aus Polyvinylbutyral, Methanol und Natriumchlorid vor (vergleiche [9]).

In einer Ausgestaltung umfasst die Herstellung des ersten Sensors das Aufbringen des ionenselektiven Materials in Form eines lonophors (beispielsweise Nonactin,Valinomycin, Natriumionophor) auf eine Arbeitselektrode. Dabei ist in einer Ausgestaltung eine Mischung mit einer Matrix (netzwerkbildende Materialien, insbesondere Polymere und vorzugsweise Polyvinylbutyral, PVB, und Polyvinylchlorid, PVC) vorgesehen.

In einer Ausgestaltung wird die erforderliche Menge des lonophors möglichst reduziert, um so einen kostengünstigen Sensors zu realisieren, der auch als Wegwerfprodukt verstanden werden kann.

Eine Ausgestaltung besteht darin, dass die Vorrichtung eine Ausgabevorrichtung aufweist. Dabei ist die Ausgabevorrichtung derartig ausgestaltet, das von der Auswerteeinheit erzeugte skalare Maß auszugeben. Die Ausgabevorrichtung übermittelt das Maß in einer Ausgestaltung an eine Anzeigeeinheit, die z. B. von einem Sportler als Uhr getragen werden kann. Alternativ oder ergänzend wird das Maß an einen Computer oder eine ähnliche Verarbeitungseinheit übermittelt.

In einer Ausgestaltung ist vorgesehen, dass die Ausgabevorrichtung derartig ausgestaltet ist, das Maß per Funk auszugeben.

Eine Ausgestaltung besteht darin, dass die Vorrichtung einen Datenspeicher aufweist. Dabei ist die Auswerteeinheit derartig ausgestaltet, basierend auf dem Messwert des ersten Sensors und mindestens dem Messwert des zweiten Sensors sowie ausgehend von in dem Datenspeicher hinterlegten Daten das skalare Maß zu erzeugen. Die Daten im Datenspeicher beziehen sich beispielsweise auf Referenzwerte oder Toleranzbereiche, innerhalb derer sich Messwerte befinden können. Alternativ oder ergänzend beziehen sich die Daten auf Kalibrierungsmessungen. Die Daten beziehen sich dabei zumindest auf Kombinationen von zwei gemessenen Werten mit dem Maß für die Belastung und beziehen sich ergänzend in einer Ausgestaltung auf eine Kombination von mindestens drei gemessenen Werten mit dem Maß für die Belastung.

In einer Ausgestaltung ist vorgesehen, dass die Vorrichtung eine Energiequelle aufweist. Dabei ist die Energiequelle derartig ausgestaltet, die Auswerteeinheit mit Energie zu versorgen. Die Energiequelle ist dabei in einer Ausgestaltung eine Batterie oder ein Akkumulator. In einer weiteren Ausgestaltung erlaubt die Energiequelle ein "Energy Harvesting", indem Bewegungen oder Wärmeenergie des zu untersuchenden Lebewesens in elektrische Energie umgewandelt werden.

Weiterhin bezieht sich die Beschreibung auf ein Verfahren zur Uberwachung eines Zustands eines Lebewesens. Dabei umfasst das Verfahren, dass ein Messwert basierend auf einem Anteil von Ammoniak im Schweiß des Lebewesens nicht-invasiv erzeugt wird, dass ein Messwert bezüglich der Atmung oder der Herztätigkeit des Lebewesens erzeugt wird sowie dass aus den Messwerten - für Ammoniak und Atmung bzw. Herztätigkeit -ein skalares Maß für eine Belastung des Lebewesens ermittelt wird.

Die oben beschriebenen Ausgestaltungen der Vorrichtung lassen sich dabei auch durch das Verfahren realisieren, so dass die Ausgestaltungen und Ausführungen entsprechend auch für das Verfahren gelten. Daher wird auf Wiederholungen verzichtet.

Im Einzelnen gibt es eine Vielzahl von Möglichkeiten, die erfindungsgemäße Vorrichtung und das Verfahren auszugestalten und weiterzubilden. Dazu wird verwiesen einerseits auf die Patentansprüche, andererseits auf die folgende Beschreibung von Ausführungsbeispielen in Verbindung mit der Zeichnung. Es zeigen:
- Fig. 1: eine schematische Darstellung eines Lebewesens mit einer erfindungsgemäßen Vorrichtung gemäß einer ersten Variante,
- Fig. 2: einen Teil einer erfindungsgemäßen Vorrichtung gemäß einer zweiten Variante als Blockschaltbild,
- Fig. 3: einen schematischen Schnitt durch einen ersten Sensor und
- Fig. 4: eine Draufsicht auf einen ersten Sensor als Teil einer erfindungsgemäßen Vorrichtung.

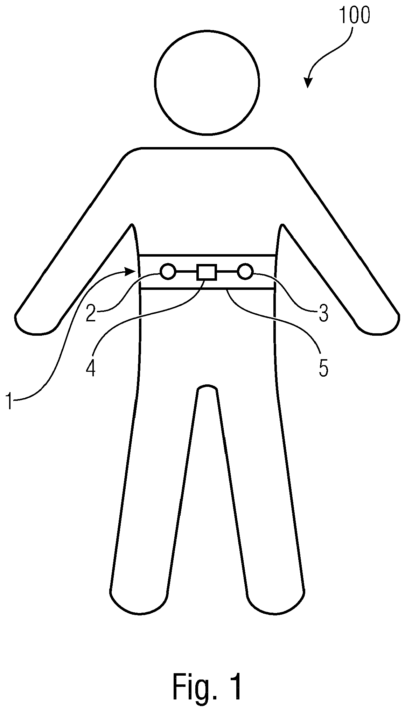

In der Fig. 1 ist schematisch eine Anwendung der erfindungsgemäßen Vorrichtung 1 dargestellt. Die beispielhafte Vorrichtung 1 wird dabei als eine Art von Gurt auf der Herzhöhe von dem Lebewesen 100 getragen, dessen Zustand durch die Vorrichtung 1 überwacht werden soll.

Die Vorrichtung 1 verfügt über einen ersten Sensor 2 sowie einen zweiten Sensor 3, die jeweils Messwerte erzeugen. Die Messwerte werden direkt vor Ort von der Auswertevorrichtung 4 ausgewertet.

Dabei dient in der dargestellten Variante der erste Sensor 2 der Untersuchung des Ammoniakanteils im Schweiß des Lebewesens 100. Der zweite Sensor 3 gibt einen Messwert bezüglich der Herztätigkeit aus. Je nach Ausgestaltung handelt es sich dabei um einen Wert für den Herzschlag, den Puls oder z. B. um einen Blutdruckwert oder einen Wert zur Atmung.

Der erste Sensor 2, der zweite Sensor 3 sowie die Auswertevorrichtung 4 befinden sich in einer Trägervorrichtung 5, die hier - wie bereits erwähnt - als eine Art Gurt ausgestaltet ist. Die elektronischen Komponenten 2, 3, 4 befinden sich dabei teilweise in Taschen der Trägervorrichtung 5 und sind teilweise direkt in dem Gewebe eingebracht. Alternativ ist eine Befestigung mittels Klettverschluss vorgesehen. Die in der Abbildung angedeutete Verkabelung befindet sich dabei ebenfalls in dem Gewebe. Die beiden der eigentlichen Messung dienenden Komponenten: erster Sensor 2 und zweiter Sensor 3 sind dabei derartig ausgestaltet und in der Trägervorrichtung 5 angeordnet, so dass sie möglichst nah am Lebewesen 100 befindlich sind.

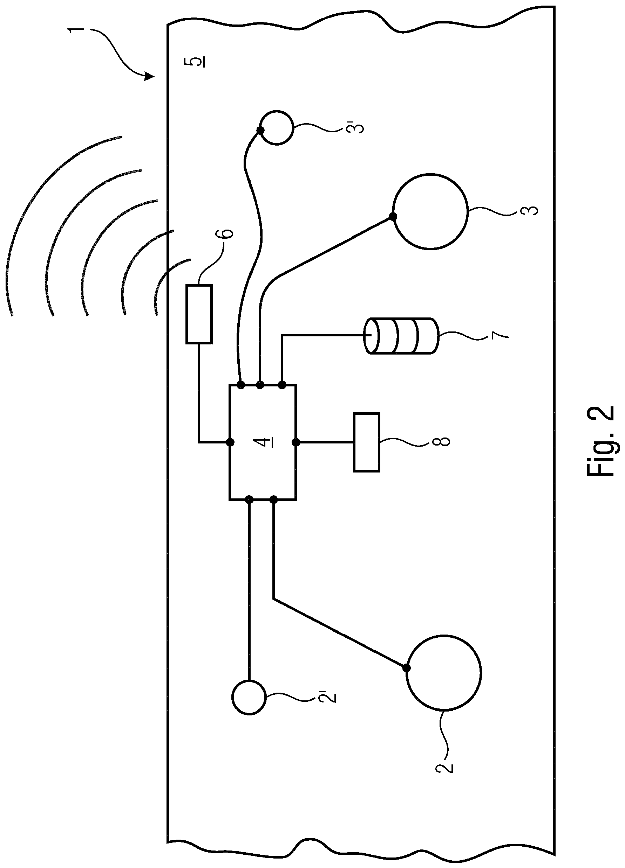

In der Fig. 2 sind schematisch Bestandteile einer weiteren Ausgestaltung der erfindungsgemäßen Vorrichtung 1 dargestellt. Die Trägervorrichtung 5 ist dabei nur angedeutet.

Vorhanden sind hier zwei erste Sensoren 2, 2', die jeweils der Ammoniakmessung dienen und die an unterschiedlichen Orten befestigt sind. Daher erlauben sie einen ortsbezogene Messung des Ammoniakgehalts.

Der - hier als solcher bezeichnete - erste zweite Sensor 3 ist an einem dritten Ort angeordnet und erlaubt in der dargestellten Variante die Messung der Herztätigkeit, z. B. den Puls über entsprechend vorgesehene Elektroden. Der zweite zweite Sensor 3' dient der Messung der Atmung. Der zweite zweite Sensor 3' ist dabei in einer Variante als Bewegungssensor ausgestaltet und erlaubt in einer weiteren Variante die Ermittlung der Laufzeit von Signalen, um berührungslos die Atmung zu überwachen und die Atemrate zu ermitteln.

Die Messwerte der zwei ersten Sensoren 2, 2' sowie der zwei zweiten Sensoren 3, 3' werden kabelgebunden der Auswertevorrichtung 4 zugeführt, die daraus ein skalares Maß erzeugt. Dieses Maß erlaubt eine Aussage über den aktuellen Zustand, insbesondere über die Belastung des Lebewesens und wird über eine Ausgabevorrichtung 6 per Funk ausgegeben. Das Maß wird beispielsweise als ein Messergebnis zu einer - hier nicht dargestellten - Anzeigeeinheit übermittelt.

In der dargestellten Ausgestaltung wird durch die Auswertevorrichtung 4 eine Bewertung der Messwerte vorgenommen, indem die aktuellen Messwerte mit in einem Datenspeicher 7 hinterlegten Referenzdaten in Beziehung gesetzt werden. So finden sich beispielweise Referenzdaten bezüglich der Bereiche, in denen der Ammoniakmesswert sich in Abhängigkeit von der Atemrate und dem Puls befinden sollte. Das Maß besteht daher beispielsweise in einer Art von Ampelsignal, d. h. rot, gelb oder grün, insofern die Referenzdaten entsprechende Wertebereiche für die Messwerte umfassen.

Die Energieversorgung geschieht über eine Energiequelle 8, bei der es sich in dem gezeigten Beispiel um eine Knopfzelle handelt. Die Energiequelle 8 ist dabei mit der Auswertevorrichtung 4 verbunden, die wiederum die ersten Sensoren 2, 2' sowie die zweiten Sensoren 3, 3' mit der für die Messungen notwendigen Energie versorgt.

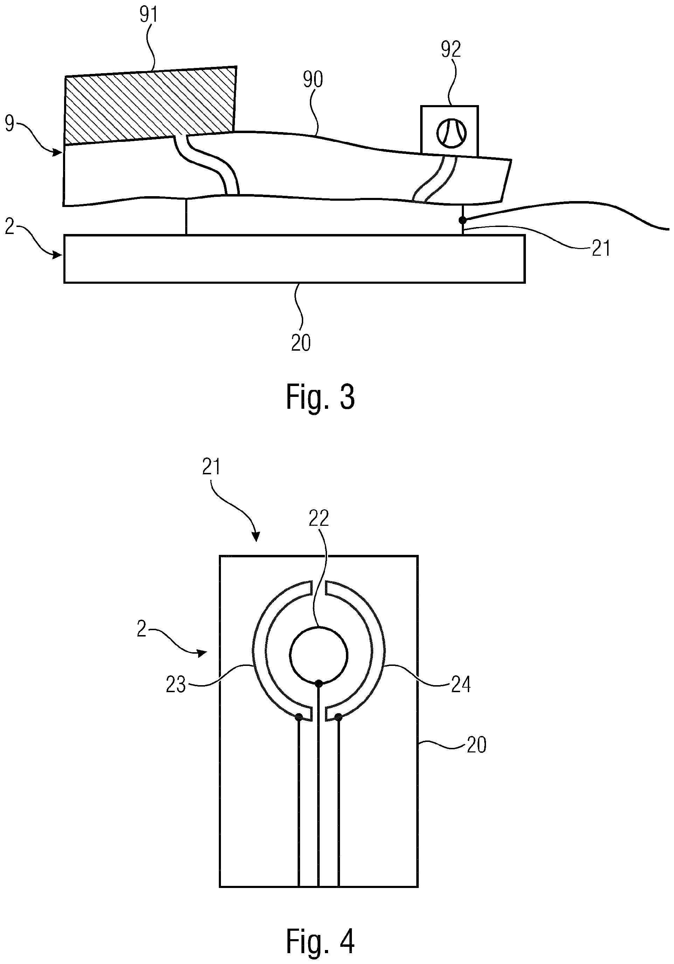

Die Fig. 3 zeigt einen Schnitt durch eine schematische Darstellung eines ersten Sensors 2 zur Messung eines Ammoniakanteils. Der erste Sensor 2 verfügt dabei über eine auf einer Folie 20 gedruckte ionenselektive Elektrode 21 (vergleiche die folgende Fig. 4), die über eine angedeutete Leitung mit der - hier nicht gezeigten - Auswertevorrichtung verbunden ist. Die Folie 20 ist dabei so flexibel und vorzugsweise auch hautfreundlich, dass sie die Anwendung unter der Belastung durch die Bewegungen des Lebewesens und den unmittelbaren Hautkontakt erlaubt.

Im dargestellten Fall befindet sich auf der von der Folie 20 abgewandten Seite der ionenselektive Elektrode 21 eine Transportvorrichtung 9, die hier sowohl den Transport des Schweißes zum ersten Sensor 2 als auch den Abtransport fort vom ersten Sensor 2 realisiert.

Dabei verfügt die Transportvorrichtung 9 in dem gezeigten Beispiel über drei unterschiedliche Komponenten 90, 91, 92:
Zum einen ist dies eine mit Kanälen oder Kapillaren versehene Komponente 90, die hier direkt auf der ionenselektiven Elektrode 21 angeordnet ist.

An einem von der ionenselektiven Elektrode 21 abgewandten Ende eines Kanals befindet sich eine Komponente 91, die ein saugfähiges Material aufweist. Die Saugfähigkeit ist dabei beispielsweise über die Größe von Poren des Materials gegeben.

An einem Ausgang eines weiteren Kanals befindet sich als dritte Komponente eine Pumpvorrichtung 92, die die Körperflüssigkeit Schweiß nach der Messung mit dem ersten Sensor 2 abtransportiert. Alternativ befindet sich auch an diesem Ausgang ein saugfähiges Material, das sich z. B. durch seine Porenausgestaltung von dem erstgenannten Material 91 unterscheidet.

Der erste Sensor 2 und die Transportvorrichtung 9 sind hier mehrteilig ausgestaltet und sind in einer alternativen Ausgestaltung einstückig ausgeführt.

Die Fig. 4 erlaubt einen Blick auf die ionenselektive Elektrode 21 des ersten Sensors 2, die auf einer flexiblen Folie 20 aufgebracht ist. Über die ionenselektive Elektrode 21 wird die Konzentration eines Ions ermittelt, das ein Maß für den Ammoniakanteil am Schweiß des zu untersuchenden Lebewesens. Hier handelt es sich vorzugsweise um das Ammonium-lon NH₄⁺.

Hierfür ist eine kreisförmige ionenselektive Membran 22 vorhanden, die umgeben ist von zwei halbkreisförmigen Elektroden in Form einer Referenzelektrode 23 und eine Gegenelektrode 24. Die ionenselektive Membran 22 trennt dabei den Schweiß als Messmedium von der Elektrodenanordnung des ersten Sensors 2. Die Membran 22 ist entsprechend so eingestellt, dass vorzugweise nur die gewünschten Ionen passieren können.

An der Stelle der ionenselektiven Membran 22 befindet sich noch eine Arbeitselektrode, deren elektrische Kontaktierung über eine Leitung dargestellt ist.

Aus der gemessenen elektrischen Spannung wird anschließend von der Auswerteinheit auf die Konzentration der Ionen und z. B. an Hand von Kalibrierungsdaten auf die Ammoniakkonzentration geschlossen.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

### Referenzen

[1] S. Imani et al., "A wearable chemical-electrophysiological hybrid biosensing system for real-time health and fitness monitoring", Nat. Commun. 7:11650, doi: 10.1038/ncomms11650, 2016.
[2] W. Gao et al., "Fully integrated wearable sensor arrays for multiplexed in situ perspiration analysis", Nature, 2016, S. 509 - 514.
[3] K. Mitsubayashi et al., "Analysis of metabolites in sweat as a measure of physical condition", Analytica Chimica Acta, 1994, S. 27 - 34.
[4] I. Alvear-Ordenes et al., "Sweat lactate, ammonia, and urea in rugby players", International Journal of Sports Medicine, 2005, S. 632 - 637.
[5] F. Meyer et al., "Effect of age and gender on sweat lactate and ammonia concentrations during exercise in the heat," Brazilian Journal of Medical and Biological Research, 2007, S. 135 - 143.
[6] D. Czarnowski et al., "Plasma ammonia is the principal source of ammonia in sweat," European Journal of Applied Physiology, 1992, S. 135 - 137.
[7] J. M. Lowenstein, "The purine nucleotide cycle revised," International Journal of Sports Medicine, 1990, S. 37 - 46.
[8] H. Schulz et al., "Ammoniak in der Leistungsdiagnostik", Deutsche Zeitschrift für Sportmedizin, 2001, S. 107 - 108.
[9] T. Guinovart et al., "Potentiometric sensors using cotton yarns, carbon nanotubes and polymeric membranes", Analyst, 2013, 5208 - 5215.

## Patentansprüche

1. Vorrichtung (1) zur Überwachung eines Zustands eines Lebewesens (100), mit mehreren zur Schweißanalyse ausgebildeten Sensoren (2), einem zweiten Sensor (3) und einer Auswertevorrichtung (4),
wobei ein zur Schweißanalyse ausgebildeter Sensor (2) derartig ausgestaltet ist, basierend auf einem Anteil von Ammoniak im Schweiß des Lebewesens (100) nicht-invasiv einen Messwert zu erzeugen, und ein weiterer zur Schweißanalyse ausgebildeter Sensor (2) gleich ausgeführt ist oder ein weiterer zur Schweißanalyse ausgebildeter Sensor (2) derartig ausgestaltet ist, einen Messwert für einen Anteil einer von Ammoniak verschiedenen Komponente im Schweiß des Lebewesens (100) zu erzeugen,
wobei die zur Schweißanalyse ausgebildeten Sensoren (2) an unterschiedlichen Positionen mit unterschiedlichen Muskelpartien des Lebewesens positionierbar sind,
wobei die zur Schweißanalyse ausgebildeten Sensoren (2) jeweils eine auf einer Folie (20) gedruckte ionenselektive Elektrode (21), eine ionenselektive Membran (22), ein ionenselektives Material in Form eines lonophors, eine Referenzelektrode (23) und eine Gegenelektrode (24) in einer Mischung mit netzwerkbildenden Materialien aufweisen,
wobei der zweite Sensor (3) derartig ausgestaltet ist, einen Messwert bezüglich der Atmung oder der Herztätigkeit des Lebewesens (100) zu erzeugen, wobei die zur Schweißanalyse ausgebildeten Sensoren (2) und der zweite Sensor (3) in einer an einem Körper des Lebewesens (100) tragbaren Trägervorrichtung (5) angeordnet sind,
wobei die Auswertvörrichtung (4) per Funk mit den zur Schweißanalyse ausgebildeten Sensoren (2) und dem zweiten Sensor (3) verbunden ist, und
wobei die Auswertevorrichtung (4) derartig ausgestaltet ist, basierend auf den Messwerten der zur Schweißanalyse ausgebildeten Sensoren (2) und dem Messwert des zweiten Sensors (3) ein skalares Maß für eine Belastung des Lebewesens (100) zu erzeugen,
wobei die Auswertevorrichtung einen Messwert zumindest eines zur Schweißanalyse ausgebildeten Sensors (2) mit einem Messwert des zweiten Sensors korreliert.

2. Vorrichtung (1) nach Anspruch 1,
wobei die zur Schweißanalyse ausgebildeten Sensoren (2), der zweite Sensor (3) und die Auswertevorrichtung (4) in der Trägervorrichtung (5) angeordnet sind.

3. Vorrichtung (1) nach Anspruch 1 oder 2,
wobei die Vorrichtung (1) mehrere zweite Sensoren (3, 3') aufweist, und wobei ein zweiter Sensor (3') einen Messwert bezüglich der Atmung und ein anderer zweiter Sensor (3) einen Messwert bezüglich der Herztätigkeit erzeugt.

4. Vorrichtung (1) nach einem der Ansprüche 1 bis 3,
wobei die Vorrichtung (1) mindestens eine Transportvorrichtung (9) aufweist, und wobei die Transportvorrichtung (9) derartig ausgestaltet ist, den Schweiß hin zu dem zur Schweißanalyse ausgebildeten Sensor (2) und/oder fort von dem zur Schweißanalyse ausgebildeten Sensor (2) zu transportieren.

5. Vorrichtung (1) nach Anspruch 4,
wobei die Transportvorrichtung (9) mindestens eine mit mindestens einem Kanal versehene Komponente (90) aufweist.

6. Vorrichtung (1) nach Anspruch 4 oder 5,
wobei die Transportvorrichtung (9) mindestens eine ein saugfähiges Material aufweisende Komponente (91) aufweist.

7. Vorrichtung (1) nach einem der Ansprüche 4 bis 6,
wobei die Transportvorrichtung (9) mindestens eine Pumpvorrichtung (92) aufweist.

8. Vorrichtung (1) nach einem der Ansprüche 1 bis 7,
wobei die Vorrichtung (1) eine Ausgabevorrichtung (6) aufweist, und wobei die Ausgabevorrichtung (6) derartig ausgestaltet ist, das von der Auswerteeinheit (4) erzeugte skalare Maß auszugeben.

9. Vorrichtung (1) nach Anspruch 8,
wobei die Ausgabevorrichtung (6) derartig ausgestaltet ist, das skalare Maß per Funk auszugeben.

10. Vorrichtung (1) nach einem der Ansprüche 1 bis 9,
wobei die Vorrichtung (1) einen Datenspeicher (7) aufweist, und
wobei die Auswerteeinheit (4) derartig ausgestaltet ist, basierend auf dem Messwert des zur Schweißanalyse ausgebildeten Sensors (2) und dem Messwert des zweiten Sensors (3) sowie ausgehend von in dem Datenspeicher (7) hinterlegten Daten das skalare Maß zu erzeugen.

11. Vorrichtung (1) nach einem der Ansprüche 1 bis 10,
wobei die Vorrichtung (1) eine Energiequelle (8) aufweist, und
wobei die Energiequelle (6) derartig ausgestaltet ist, die Auswerteeinheit (4) mit Energie zu versorgen.

## Claims

1. Device (1) for monitoring a state of a living being (100),
comprising several sensors (2) implemented for sweat analysis, a second sensor (3) and an evaluation device (4),
wherein a sensor (2) implemented for sweat analysis is configured in such a way as to non-invasively generate a measurement value based on a content of ammonia in the sweat of the living being (100), and a further sensor (2) implemented for sweat analysis is embodied identically, or a further sensor (2) implemented for sweat analysis is configured in such a way as to generate a measurement value for a content of a component in the sweat of the living being (100) other than ammonia,
wherein the sensors (2) implemented for sweat analysis may be positioned at different positions with different muscle groups of the living being,
wherein the sensors (2) implemented for sweat analysis each comprise an ion-selective electrode (21) printed on a film (20), an ion-selective membrane (22), an ion-selective material in the form of an ionophore, a reference electrode (23) and a counter electrode (24) in a mixture with network-forming materials,
wherein the second sensor (3) is configured in such a way as to generate a measurement value with respect to the respiration or the cardiac activity of the living being (100),
wherein the sensors (2) implemented for sweat analysis and the second sensor (3) are arranged in a carrier device (5) wearable at a body of the living being (100), and
wherein the evaluation device (4) is configured in such a way as to generate a scalar measure of a stress of the living being (100) based on the measurement values of the sensors (2) implemented for sweat analysis and the measurement value of the second sensor (3),
wherein the evaluation device correlates a measurement value of at least one sensor (2) implemented for sweat analysis with a measurement value of the second sensor.

2. Device (1) according to claim 1,
wherein the sensors (2) implemented for sweat analysis, the second sensor (3) and the evaluation device (4) are arranged in the carrier device (5).

3. Device (1) according to claim 1 or 2,
wherein the device (1) comprises several second sensors (3, 3'), and
wherein one second sensor (3') generates a measurement value with respect to the respiration and another second sensor (3) generates a measurement value with respect to the cardiac activity.

4. Device (1) according to any one claims 1 to 3,
wherein the device (1) comprises at least one transport device (9), and
wherein the transport device (9) is configured in such a way as to transport the sweat to the sensor (2) implemented for sweat analysis and/or away from the sensor (2) implemented for sweat analysis.

5. Device (1) according to claim 4,
wherein the transport device (9) comprises at least one component (90) provided with at least one channel.

6. Device (1) according to claim 4 or 5,
wherein the transport device (9) comprises at least one component (91) comprising an absorbent material.

7. Device (1) according to any one claims 4 to 6,
wherein the transport device (9) comprises at least one pumping device (92).

8. Device (1) according to any one claims 1 to 7,
wherein the device (1) comprises an output device (6), and
wherein the output device (6) is configured in such a way as to output the scalar measure generated by the evaluation unit (4).

9. Device (1) according to claim 8,
wherein the output device (6) is configured in such a way as to output the scalar measure by radio.

10. Device (1) according to any one claims 1 to 9,
wherein the device (1) comprises a data memory (7), and
wherein the evaluation unit (4) is configured in such a way as to generate the scalar measure based on the measurement value of the sensor (2) implemented for sweat analysis and the measurement value of the second sensor (3) as well as based on data stored in the data memory (7).

11. Device (1) according to any one claims 1 to 10,
wherein the device (1) comprises an energy source (8), and
wherein the energy source (6) is configured in such a way as to supply energy to the evaluation unit (4).

## Revendications

1. Dispositif (1) de surveillance d'un état d'un être vivant (100),
avec plusieurs capteurs (2) conçus pour l'analyse de la sueur, un deuxième capteur (3) et un dispositif d'évaluation (4),
dans lequel un capteur (2) conçu pour l'analyse de la sueur est conçu de sorte à générer, sur base d'une part d'ammoniac dans la sueur de l'être vivant (100), de manière non invasive une valeur de mesure, et un autre capteur (2) conçu pour l'analyse de la sueur est conçu de manière identique ou un autre capteur (2) conçu pour l'analyse de la sueur est conçu de sorte à générer une valeur de mesure pour une part d'un composant différent de l'ammoniac dans la sueur de l'être vivant (100),
dans lequel les capteurs (2) conçus pour l'analyse de la sueur peuvent être positionnés à différentes positions avec différentes parties musculaires de l'être vivant,
dans lequel les capteurs (2) conçus pour l'analyse de la sueur présentent, chacun, une électrode sélective d'ions (21) imprimée sur un film (20), une membrane sélective d'ions (22), un matériau sélectif d'ions sous forme d'un ionophore, une électrode de référence (23) et une contre-électrode (24) dans un mélange avec des matériaux formant un réseau,
dans lequel le deuxième capteur (3) est conçu de sorte à générer une valeur de mesure en ce qui concerne la respiration ou l'activité cardiaque de l'être vivant (100),
dans lequel les capteurs (2) conçus pour l'analyse de la sueur et le deuxième capteur (3) sont disposés dans un dispositif porteur (5) portable sur un corps de l'être vivant (100),
dans lequel le dispositif d'évaluation (4) est connecté par radio aux capteurs (2) conçus pour l'analyse de la sueur et au deuxième capteur (3), et
dans lequel le dispositif d'évaluation (4) est conçu de sorte à générer, sur base des valeurs de mesure des capteurs (2) conçus pour l'analyse de la sueur et de la valeur de mesure du deuxième capteur (3), une mesure scalaire d'un stress de l'être vivant (100),
dans lequel le dispositif d'évaluation corrèle une valeur de mesure d'au moins un capteur (2) conçu pour l'analyse de la sueur avec une valeur de mesure du deuxième capteur.

2. Dispositif (1) selon la revendication 1,
dans lequel les capteurs (2) conçus pour l'analyse de la sueur, le deuxième capteur (3) et le dispositif d'évaluation (4) sont disposés dans le dispositif porteur (5).

3. Dispositif (1) selon la revendication 1 ou 2,
dans lequel le dispositif (1) présente plusieurs deuxièmes capteurs (3, 3'), et
dans lequel un deuxième capteur (3') génère une valeur de mesure en ce qui concerne la respiration et un autre deuxième capteur (3) génère une valeur de mesure en ce qui concerne l'activité cardiaque.

4. Dispositif (1) selon l'une des revendications 1 à 3,
dans lequel le dispositif (1) présente au moins un dispositif porteur (9), et
dans lequel le dispositif porteur (9) est conçu de sorte à transporter la sueur vers le capteur (2) conçu pour l'analyse de la sueur et/ou en partant du capteur (2) conçu pour l'analyse de la sueur.

5. Dispositif (1) selon la revendication 4,
dans lequel le dispositif porteur (9) présente au moins un composant (90) pourvu d'au moins un canal.

6. Dispositif (1) selon la revendication 4 ou 5,
dans lequel le dispositif porteur (9) présente au moins un composant (91) présentant un matériau absorbant.

7. Dispositif (1) selon l'une des revendications 4 à 6,
dans lequel le dispositif porteur (9) présente au moins un dispositif de pompage (92).

8. Dispositif (1) selon l'une des revendications 1 à 7,
dans lequel le dispositif (1) présente un dispositif de sortie (6), et
dans lequel le dispositif de sortie (6) est conçu de sorte à sortir la mesure scalaire générée par l'unité d'évaluation (4).

9. Dispositif (1) selon la revendication 8,
dans lequel le dispositif de sortie (6) est conçu de sorte à sortir la mesure scalaire par radio.

10. Dispositif (1) selon l'une des revendications 1 à 9,
dans lequel le dispositif (1) présente une mémoire de données (7), et
dans lequel l'unité d'évaluation (4) est conçue de sorte à générer la mesure scalaire sur base de la valeur de mesure du capteur (2) conçu pour l'analyse de la sueur et de la valeur de mesure du deuxième capteur (3) ainsi qu'en partant des données mémorisées dans la mémoire de données (7).

11. Dispositif (1) selon l'une des revendications 1 à 10,
dans lequel le dispositif (1) présente une source d'énergie (8), et
dans lequel la source d'énergie (6) est conçue de sorte à alimenter l'unité d'évaluation (4) en énergie.
